# EUROPÄISCHE PATENTSCHRIFT

(11) **EP 1 199 989 B1**
(45) Veröffentlichungstag und Bekanntmachung des Hinweises auf die Patenterteilung: **15.10.2003**
(21) Anmeldenummer: 00956327.1
(22) Anmeldetag: 28.07.2000
(51) Int. Cl.: A61B 17/02

(54) **MEDIZINISCHES INSTRUMENT ZUR SCHAFFUNG EINES HOHLRAUMS FUR EINEN ENDOSKOPISCHEN EINGRIFF**
MEDICAL INSTRUMENT AND METHOD FOR CREATING A CAVITY FOR AN ENDOSCOPIC INTERVENTION
INSTRUMENT MEDICAL POUR CREER UNE CAVITE AFIN DE PRATIQUER UNE INTERVENTION ENDOSCOPIQUE

(30) Priorität: 05.08.1999 DE 19937043
(43) Veröffentlichungstag der Anmeldung: 02.05.2002
(73) Patentinhaber: Karl Storz GmbH & Co. KG, 78532 Tuttlingen (DE)
(72) Erfinder: LE HUEC, Jean, Charles, F-33600 Pessac (FR); LAFOND, Christophe, 03700 Bellerive sur Allier (FR)
(74) Vertreter: Hofmeister, Frank Horst
(86) Internationale Anmeldenummer: EP0007269
(87) Internationale Veröffentlichungsnummer: WO01010309

(56) Entgegenhaltungen:
- EP-A- 0 512 729
- EP-A- 0 705 566
- DE-A- 19 605 615
- DE-C- 4 318 950
- DE-U- 8 216 373
- US-A- 4 909 789
- US-A- 5 632 746

## Beschreibung

Die Erfindung betrifft ein medizinisches Instrument zur Schaffung eines Hohlraums für einen endoskopischen Eingriff in einem menschlichen oder tierischen Körper, mit einer in eine künstliche Körperöffnung einsetzbaren hohlzylindrischen Trokarhülse und mit einer in diese Trokarhülse einziehbaren und aus dieser ausschiebbaren Spreizvorrichtung, wobei die Trokarhülse aus zwei koaxial und mit Abstand zueinander angeordneten Hülsen besteht.

Üblicherweise wird bei endoskopischen Eingriffen beispielsweise der Bauchraum mit einem geeigneten Gas aufgepumpt, um einen ausreichenden Arbeitsraum für den Operateur zu schaffen. Der Zugang für die medizinischen Instrumente wird dabei über ein oder mehrere Trokare mit Ventil ermöglicht. Diese Art von endoskopischen Eingriffen hat sich in der Praxis zwar durchaus bewährt, jedoch ist stets auf eine gute Abdichtung im Bereich der Trokare zu achten und für eine gleichmäßige Druckregelung und Drucküberwachung zu sorgen.

Neben dieser Operationsmethode, bei der der Hohlraum für den Eingriff mittels eines Gases geschaffen wird, sind verschiedene medizinische Instrumente bekannt, um auf mechanischem Weg für den ausreichenden Arbeitsraum zu sorgen. Die Verwendung mechanischer Spreizvorrichtungen hat dabei gegenüber der Verwendung des Gases den weiteren Vorteil, daß nicht nur ein Hohlraum für die Operation geschaffen wird, sondern auch ganz gezielt einzelne Organe oder Gewebeteile aus dem Operationsgebiet herausgedrückt werden können.

Ein gattungsgemäßes medizinisches Instrument ist aus der DE-A-196 05 615 bekannt. Bei diesem bekannten Instrument besteht die Spreizvorrichtung aus zwei Federblättern, in deren mittlerem Bereich eine Abwinklung derart ausgebildet ist, daß sich die Federarme bogenförmig von der Trokarhülse nach außen biegen, sobald die an der Innenhülse det Trokarhülse festgelegten Federarme aus der Außenhülse der Trokarhülse herausgeschoben werden. In der gespreizten Arbeitsstellung der Federarme ist zwischen den beiden Federarmen und der Innenhülse jeweils ein Spalt ausgebildet, in den Gewebe gelangen kann, das nachfolgend beim Einziehen der lnnenhülse bzw. der Federarme eingeklemmt und verletzt werden kann.

Ein weiteres medizinisches Instrument ist aus der US 5 351 679 A bekannt. Bei diesem bekannten Instrument besteht die Spreizvorrichtung aus drei fächerförmig aufspreizbaren Metallblättern, die an ihren Enden über gelenkig angeordnete Querstege miteinander verbunden sind. Mit diesem Instrument ist es zwar möglich Gewebe aus einem Operationsgebiet herauszudrücken, die Schaffung eines freien Operationsgebiets ist mit diesem flächig wirkenden Spreizelement nicht möglich. Darüber hinaus besteht die Gefahr, daß beim Zusammenfalten der Metallblätter gewebe zwischen die Blätter gelangt und eingeklemmt wird.

Weiterhin ist aus der US-Patentschrift 5 402 772 beispielsweise eine in den Körper einführbare Vorrichtung bekannt, die im Körper mittels einer Flüssigkeit oder eines Gases aufgepumpt wird, um so das gewünschte Operationsgebiet zu schaffen. Durch die Verwendung eines einzuleitenden und zu überwachenden Druckmittels ist die Verwendung dieser bekannten Vorrichtung wieder sehr aufwendig und teuer. Darüber hinaus ist das aufpumpbare Material kaum so zu reinigen, daß es für einen zweiten Einsatz verwendet werden kann, so daß es sich hierbei um einen teuren Einmalartikel handelt.

Aus der DE-C1-43 18 950 ist weiterhin eine Gewebespreizvorrichtung zur Schaffung eines Hohlraums für einen endoskopischen Eingriff bekannt. Diese bekannte Vorrichtung besteht aus einem durch einen üblichen Trokar in den Körper einführbaren Schaft, an dessen proximalem Ende ein Griff und an dessen distalem Ende einzelne elastische Federblätter angeordnet sind, die über eine über den Griff zu betätigende Spannvorrichtung zur Bildung eines Hohlraums zusammengezogen werden können. Nachteilig bei dieser bekannten Spreizvorrichtung ist der komplizierte und vielteilige Aufbau der Spannvorrichtung, die darüber hinaus nach jedem Gebrauch ganz auseinandergenommen werden muß, um diese gründlich reinigen zu können.

Ausgehend von diesem Stand der Technik liegt der Erfindung die **Aufgabe** zugrunde, ein medizinisches Instrument zur Schaffung eines Hohlraums für einen endoskopischen Eingriff zu schaffen, daß aus wenigen Einzelteilen aufgebaut ist und eine einfache Handhabung ermöglicht.

Die Lösung dieser Aufgabenstellung ist erfindungsgemäß dadurch gekennzeichnet, daß die Spreizvorrichtung ein außerhalb der Trokarhülse angeordnetes Halteelement und wenigstens zwei Federblätter aus einem flexiblen Material aufweist, die jeweils in ihrem mittleren Abschnitt einen Bogen bilden und jeweils mit ihren beiden Enden durch die Trokarhülse verlaufend an diesem Halteelement befestigt sind und, daß die in dem Halteelement festgelegten Federblätter durch den zwischen den beiden koaxialen Hülsen ausgebildeten Spalt verlaufen.

Dieses erfindungsgemäße medizinische Instrument zeichnet sich dadurch aus, daß es aus nur wenigen Bauteilen besteht, nämlich der Trokarhülse, dem Halteelement und den Federblättern. Durch das einen Bogen bildende Umbiegen der mit ihren beiden Enden am Halteelement festgelegten Federblätter dehnen sich die Federblätter nach dem Austritt aus der Trokarhülse sofort selbsttätig auseinander, so daß auf eine Spannvorrichtung verzichtet werden kann, wie diese aus dem Stand der Technik bekannt ist. Diese erfindungsgemäße Spreizvorrichtung ermöglicht die Ausbildung eines räumlich ausgebildeten Operationshohlraums.

Gemäß einer praktischen Ausführungform der Erfindung wird vorgeschlagen, daß die Trokarhülse aus zwei koaxial mit Abstand zueinander angeordneten Hülsen besteht, wobei die in dem Halteelement festgelegten Federblätter durch den zwischen den beiden koaxialen Hülsen gebildeten Spalt verlaufen. Diese Ausgestaltungsform hat den Vorteil, daß das Instrument einteilig ausgebidet ist und sich ein Einfädeln der Spreizvorrichtung in die Trokarhülse erübrigt, da die Federblätter in der doppelwandigen Trokarhülse gelagert sind.

Um einen räumlich ausgedehnten Hohlraum zu schaffen, sind die wenigstens zwei Federblätter so versetzt zueinander an dem Halteelement angeordnet, daß die durch die Bogen aufgespannten Ebenen sich schneiden. Ein möglichst großer Hohlraum für den endoskopischen Eingriff erhält man dann, wenn zwei Federblätter um 90° versetzt zueinander am Halteelement festgelegt sind. Bei dieser Anordnung ergibt sich durch die beiden sich unter einem, Winkel von 90° schneidenden Bogen der beiden Federblätter ein kugelförmiger Hohlraum.

Gemäß einer bevorzugten Ausführungsform der Erfindung wird vorgeschlagen, daß die Spreizvorrichtung aus vier am Halteelement festgelegten Federblättern besteht, die jeweils um 45° zueinander versetzt am Halteelement festgelegt sind. Durch diese acht Arme der vier bogenförmig gebogenen Federblätter ist es besonders gut möglich, nicht zu behandelndes Gewebe aus dem Operationsgebiet herauszuhalten, gleichzeitig aber trotzdem genug Platz zwischen den einzelnen Armen der Federblätter zu lassen, um zwischen ihnen hindurch ein beispielsweise chirurgisches Instrument in den so geschaffenen Hohlraum einzuführen.

Damit sich die einzelnen Bogen der Federblätter nicht gegeneinander verschieben können, wird gemäß einer praktischen Ausführungsform der Erfindung vorgeschlagen, daß die einzelnen bogenförmig gebogenen Federblätter in ihrem Scheitelpunkt über ein gemeinsames Verbindungselement miteinander verbunden sind.

Als Material für die Federblätter wird vorzugsweise TiNi verwendet, jedoch kann auch jedes andere flexible und körperverträgliche Material verwendet werden.

Die Vielseitigkeit des erfindungsgemäßen, Instruments kann dadurch gesteigert werden, daß das Halteelement eine zentrale Öffnung aufweist, durch die ein oder mehrere medizinische Instrumente in den Körper eingeführt werden können. Dabei kann es sich beispielsweise um ein Endoskop, eine Faßzange, eine Schere, ein HF-Instrument oder ein Saug-/ Spülrohr handeln. Diese Ausführungsform ist besonders vorteilhaft, da dieses die Spreizvorrichtung eingeführte Instrument automatisch gleich in dem künstlich geschaffenen Hohlraum gelegen ist.

Gemäß einer Weiterentwicklung des erfindungsgemäßen medizinischen Instruments ist am Halteelement angreifende Arretiervorrichtung vorgesehen, um die Spreizvorrichtung in der jeweiligen in die Trokarhülse eingeführten Stellung festlegen zu können.

Weitere Merkmale und Vorteile der Erfindung ergeben sich aus der nachfolgenden Beschreibung der zugehörigen Zeichnung, in der ein Ausführungsbeispiel eines erfindungsgemäßen medizinischen Instruments zur Schaffung eines Hohlraums dargestellt ist. In der Zeichnung zeigt:
- Fig.1: eine teilweise geschnittene schematische Seitenansicht des erfindungsgemäßen medizinischen Instruments mit zusammengezogenen Federblättern,
- Fig.2: eine Ansicht gemäß Fig.1, jedoch die Federblätter in der Arbeitsstellung darstellend und
- Fig.3: eine Draufsicht auf das Instrument gemäß Fig.1 vom distalen Ende her.

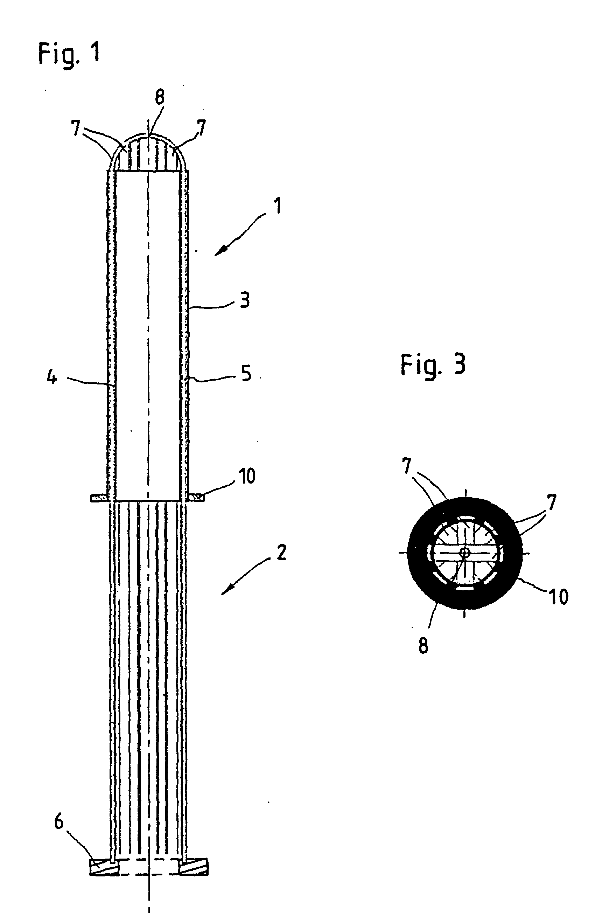

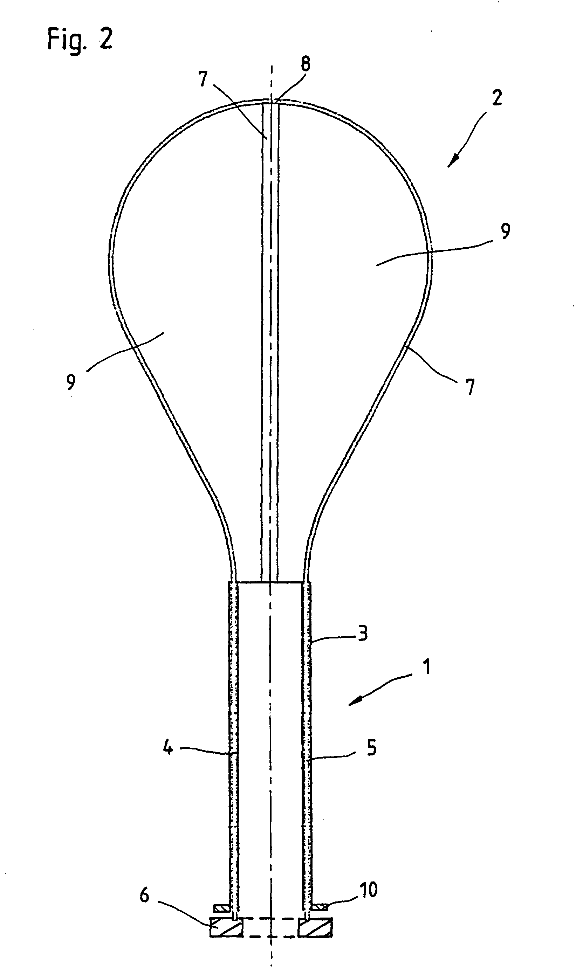

Das in den Abbildungen Fig. 1 und Fig. 2 dargestellte medizinische Instrument besteht im wesentlichen aus einer Trokarhülse 1 und einer in die Trokarhülse 1 einschiebbaren Spreizvorrichtung 2.

Beim dargestellten Ausführungsbeispiel besteht die Trokarhülse 1 aus einer Außenhülse 3 und einer koaxial in der Außenhülse 3 gelagerten Innenhülse 4, wobei Außenhülse 3 und Innenhülse 4 einen Spalt 5 bildend voneinander beabstandet sind.

Die Spreizvorrichtung 2 ihrerseits besteht aus einem am proximalen Ende angeordneten Halteelement 6 und beim dargestellten Ausführungsbeispiel vier an dem Halteelement 6 festgelegten Federblättern 7. Wie insbesondere aus Fig. 2 ersichtlich, ist jedes Federblatt 7 einen Bogen bildend mit beiden Enden in dem Halteelement 6 festgelegt und so versetzt zum nächsten Federblatt 7 angeordnet, daß sich die durch die Bogen aufgespannten Ebenen schneiden. Um ein gegenseitiges Verrutschen der sich durchgreifenden Bogen der Federblätter 7 zu verhindern, sind die Federblätter 7 in den Scheitelpunkten ihrer Bogen über ein gemeinsames Verbindungselement 8 miteinander verbunden. Die Abbildungen zeigen weiterhin, daß bei dieser Ausführungsform die Federblätter 7 ausgehend von dem Halteelement 6 durch den Spalt 5 zwischen der Innenhülse 4 und der Außenhülse 3 der Trokarhülse 1 verlaufen.

Das Arbeiten mit dem dargestellten medizinischen Instrument geschieht wie folgt:

Zum Einführen des medizinischen Instruments in einen menschlichen oder tierischen Körper wird zunächst die Trokarhülse 1 hin zum distalen Ende der Spreizvorrichtung 2 verschoben, wie dies in Fig. 1 dargestellt ist. Beim Verschieben der Trokarhülse 1 in diese Richtung werden die flexiblen Federblätter 7 so zusammengedrückt, daß der Außendurchmesser am distalen Ende der Spreizvorrichtung 2 den Außendurchmesser der Trokarhülse 1 nicht mehr übersteigt.

Anschließend wird die Trokarhülse in eine künstliche Körperöffnung eingesetzt und daraufhin die Spreizvorrichtung 2 über das Halteelement 6 durch die Trokarhülse 1 hindurchgedrückt, bis die Federblätter 7 wieder aus dem Spalt 5 der Trokarhülse 1 heraustreten und aufgrund der Federelastizität ihres Materials die in Fig. 2 dargestellte Form einnehmen, in der die versetzt zueinander am Halteelement 6 festgelegten Federblätter 7 einen kugelförmigen Hohlraum für den endoskopischen Eingriff bilden. Aus Gründen der besseren Übersichtlichkeit sind in Fig. 2 nur zwei Federblätter 7 dargestellt, obwohl die Darstellungen gemäß Fig 1 und Fig. 3 erkennen lassen, daß vier um jeweils 45° versetzt zueinander angeordnete Federblätter 7 am Halteelement 6 angeordnet sind.

Nach dem endoskopischen Eingriff, bei dem durch die zwischen den einzelnen Armen der Federblätter 7 gebildeten Fenster ein oder mehrere beispielsweise chirurgische Instrumente in den durch die Spreizvorrichtung 2 gebildeten Hohlraum eingeführt wurden, wird die Spreizvorrichtung 2 wieder über das Halteelement 6 in die Trokarhülse 1 eingezogen, so daß sich die Federblätter 7 wieder zusammenlegen, wie dies in Fig. 1 dargestellt ist. Anschließend wird die Trokarhülse 1 wieder aus der künstlichen Körperöffnung herausgezogen. Zum Ergreifen und Verschieben ist am proximalen Ende der Trokarhülse 1 ein umlaufender Rand 10 angeformt.

Das dargestellte medizinische Instrument zeichnet sich somit durch einen Aufbau aus nur wenigen Einzelteilen und eine besonders einfache Handhabung aus.

### Bezugszeichenliste

- 1: Trokarhülse
- 2: Spreizvorrichtung
- 3: Außenhülse
- 4: Innenhülse
- 5: Spalt
- 6: Halteelement
- 7: Federblatt
- 8: Verbindungselement
- 9: Fenster
- 10: Rand

## Patentansprüche

1. Medizinisches Instrument zur Schaffung eines Hohlraums für einen endoskopischen Eingriff in einem menschlichen oder tierischen Körper, mit einer in eine künstliche Körperöffnung einsetzbaren hohlzylindrischen Trokarhülse (1) und mit einer in diese Trokarhülse (1) einziehbaren und aus dieser ausschiebbaren Spreizvorrichtung (2), wobei die Trokarhülse (1) aus zwei koaxial und mit Abstand zueinander angeordneten Hülsen (3, 4) besteht, **dadurch gekennzeichnet,**
**daß** die Spreizvorrichtung (2) ein außerhalb der Trokarhülse (1) angeordnetes Halteelement (6) und wenigstens zwei Federblätter (7) aus einem flexiblen Material aufweist, die jeweils in ihrem mittleren Abschnitt einen Bogen bilden und jeweils mit ihren beiden Enden durch die Trokarhülse (1) verlaufend an diesem Halteelement (6) befestigt sind und, daß die in dem Halteelement (6) festgelegten Federblätter (7) durch den zwischen den beiden koaxialen Hülsen (3, 4) ausgebildeten Spalt (5) verlaufen.

2. Medizinisches Instrument nach Anspruch 1, **dadurch gekennzeichnet, daß** die wenigstens zwei Federblätter (7) so versetzt zueinander an dem Halteelement (6) angeordnet sind, daß sich die durch die Bogen aufgespannten Ebenen schneiden.

3. Medizinisches Instrument nach Anspruch 1 oder 2, **dadurch gekennzeichnet, daß** zwei Federblätter (7) um 90° zueinander versetzt am Halteelement (6) festgelegt sind.

4. Medizinisches Instrument nach Anspruch 1 oder 2, **dadurch gekennzeichnet, daß** die Spreizvorrichtung (2) aus vier am Halteelement (6) festgelegten Federblättern (7) besteht, die um jeweils 45° zueinander versetzt am Halteelement (6) festgelegt sind.

5. Medizinisches Instrument nach mindestens einem der Ansprüche 2 bis 4, **dadurch gekennzeichnet, daß** die einzelnen bogenförmig gebogenen Federblätter (7) in ihren Scheitelpunkten über ein gemeinsames Verbindungselement (8) miteinander verbunden sind.

6. Medizinisches Instrument nach mindestens einem der Ansprüche 1 bis 5, **dadurch gekennzeichnet, daß** die Federblätter (7) aus elastischem TiNi bestehen.

7. Medizinisches Instrument nach mindestens einem der Ansprüche 1 bis 6, **dadurch gekennzeichnet, daß** das Halteelement (6) eine zentrale Öffnung zum Einführen mindestens eines weiteren medizinischen Instruments aufweist.

8. Medizinisches Instrument nach mindestens einem der Ansprüche 1 bis 7, **gekennzeichnet durch** eine am Halteelement angreifende Arretiervorrichtung, zum Festlegen der Spreizvorrichtung (2) in der jeweiligen in die Trokarhülse (1) eingeführten Stellung.

## Claims

1. Medical instrument for creating a cavity for an endoscopic intervention in a human or animal body, with a hollow cylindrical trocar sleeve (1) which can be inserted into an artificial opening in the body, and with a spreading device (2) which can be drawn into this trocar sleeve (1) and can be pushed out from the latter, said trocar sleeve (1) being made up of two sleeves (3, 4) arranged coaxially and at a distance from one another, **characterized in that** the spreading device (2) has a retaining element (6) arranged outside the trocar sleeve (1) and at least two spring blades (7) which are made of a flexible material and which each form an arc in their middle portion and are each secured on this retaining element (6) with their two ends extending through the trocar sleeve (1), and **in that** the spring blades (7) secured in the retaining element (6) extend trough the gap (5) formed between the two coaxial sleeves (3, 4).

2. Medical instrument according to Claim 1, **characterized in that** the at least two spring blades (7) are arranged, offset in relation to one another, on the retaining element (6) in such a way that the planes spanned by the arcs intersect.

3. Medical instrument according to Claim 1 or 2, **characterized in that** two spring blades (7) are offset by 90° with respect to one another on the retaining element (6).

4. Medical instrument according to Claim 1 or 2, **characterized in that** the spreading device (2) is made up of four spring blades (7) which are secured on the retaining element (6) and which are each offset in relation to one another by 45° on the retaining element (6).

5. Medical instrument according to at least one of Claims 2 to 4, **characterized in that** the individual spring blades (7) curved in an arch shape are connected to one another at their vertices via a common connection element (8).

6. Medical instrument according to at least one of Claims 1 to 5, **characterized in that** the spring blades (7) are made of elastic TiNi.

7. Medical instrument according to at least one of Claims 1 to 6, **characterized in that** the retaining element (6) has a central opening for insertion of at least one further medical instrument.

8. Medical instrument according to at least one of Claims 1 to 7, **characterized by** a locking device engaging on the retaining element, said locking device being used to secure the spreading device (2) in the respective position to which it has been introduced into the trocar sleeve (1).

## Revendications

1. Instrument médical pour créer une cavité pour l'intervention endoscopique sur un corps humain ou animal, avec un trocart (1) cylindrique creux, pouvant être introduite dans un orifice artificiel du corps et avec un dispositif d'écartement (2) pouvant être entré dans ce trocart (1) et sorti de celui-ci, le trocart (1) se composant de deux douilles (3, 4) disposées sur le même axe et à distance l'une de l'autre,
**caractérisé en ce que**
le dispositif d'écartement (2) présente un élément de retenue (6) disposé à l'extérieur du trocart (1) et au moins deux lames de ressort (7) à base d'un matériau flexible, qui forment un arc respectivement dans leur partie centrale et sont fixées respectivement avec leurs deux extrémités en passant à travers le trocart (1) sur cet élément de retenue (6), et **en ce que** les lames de ressort (7) fixées dans l'élément de retenue (6) sont passées par la fente (5) réalisée entre les deux douilles (3, 4) coaxiales.

2. Instrument médical selon la revendication 1,
**caractérisé en ce que**
les au moins deux lames de ressort (7). sont disposées sur l'élément de retenue (6) de façon décalée l'une par rapport à l'autre, de sorte que les plans tendus par les arcs se coupent.

3. Instrument médical selon la revendication 1 ou 2,
**caractérisé en ce que**
deux lames de ressort (7) sont fixées sur l'élément de retenue (6) décalées de 90° l'une par rapport à l'autre.

4. Instrument médical selon la revendication 1 ou 2,
**caractérisé en ce que**
le dispositif d'écartement (2) se compose de quatre lames de ressorts (7) fixées sur l'élément de retenue (6), qui sont fixées sur l'élément de retenue (6) décalées à chaque fois de 45° entre elles.

5. Instrument médical selon au moins l'une quelconque des revendications 2 à 4,
**caractérisé en ce que**
les différentes lames de ressort (7) pliées en forme d'arc sont reliées entre elles en leurs sommets au moyen d'un élément de liaison (8) commun.

6. Instrument médical selon au moins l'une quelconque des revendications 1 à 6,
**caractérisé en ce que**
les lames de ressort (7) sont à base de TiNi élastique.

7. Instrument médical selon au moins l'une quelconque des revendications 1 à 6,
**caractérisé en ce que**
l'élément de retenue (6) présente une ouverture centrale pour l'introduction d'au moins un autre instrument médical.

8. Instrument médical selon au moins l'une quelconque des revendications 1 à 7,
**caractérisé par** un dispositif d'arrêt s'appliquant sur l'élément de retenue, pour la fixation du dispositif d'écartement (2) dans la position respective mise en place dans le trocart (1).
